# EUROPEAN PATENT APPLICATION

(11) **EP 4 006 389 A1**
(43) Date of publication of application: **01.06.2022**
(21) Application number: 21209992.3
(22) Date of filing: 23.11.2021
(51) Int. Cl.: F16J 15/3284, A61M 16/00

(54) **SEAL**

(30) Priority: 27.11.2020 GB 202018758
(71) Applicant: Cam Lock Limited, Aldershot, Hampshire GU12 4UH (GB)
(72) Inventor: GRIFFITHS, Joseph Anthony, Aldershot, GU12 4UH (GB)
(74) Representative: Brown, Alexander Edward

(57) **Abstract**

A seal for a ventilator piston. The seal is configured to be secured to a ventilator piston and received within a piston chamber of a ventilator to provide a slidable seal against an inner wall of the piston chamber. The seal comprises a deformable body comprising a first material, and a layer of a friction-reducing material provided on at least a portion of the deformable body and configured to provide slidable sealing contact with the inner wall of the piston chamber. The first material and the friction-reducing are different. Also provided is a ventilator comprising such a seal, and a method of manufacturing such a seal.

## Description

### Technical Field

The present disclosure relates to a seal for a ventilator piston, a ventilator comprising such a seal, and a method of manufacturing a seal for a ventilator piston.

### Background

A ventilator is a machine that mechanically assists a patient to breathe. Typically a ventilator comprises a piston tube and a piston attached to a bellows or diaphragm disposed in the piston tube. The ventilator moves breathable air into and out of a patient's lungs. In some instances a higher ventilator pressure can be required for providing breathable air to a patient's lungs, such as where a patient's lungs comprise restrictions. Some known bellows can be prone to inflate when a higher pressure is applied. Excess inflation of the bellows can defeat the object of applying the increased pressure and can fail to get the required air under the desired pressure into the patient's lungs. Therefore, there remains a need for developments in this field.

### Summary of the Invention

According to the present disclosure there is provided a seal for a ventilator piston, the seal configured to be secured to a ventilator piston and received within a piston chamber of a ventilator to provide a slidable seal against an inner wall of the piston chamber, the seal comprising a deformable body comprising a first material; and a layer of a friction-reducing material provided on at least a portion of the deformable body and configured to provide slidable sealing contact with the inner wall of the piston chamber; wherein the first material and the friction-reducing material are different.

The deformable body may comprise a substantially planar component.

The seal may comprise a laminar construction and opposing first and second surfaces, and the layer of friction-reducing material may substantially cover at least one or part of the first and second surfaces.

The seal may further comprise a peripheral wall and the layer of friction-reducing material may extend at least partially over the peripheral wall. The layer of friction-reducing material may extend entirely over the peripheral wall between opposite sides of the deformable body.

The peripheral wall may taper outwardly from a region of one of the first surface and the second surface, to the other of the first surface and the second surface. The peripheral wall may taper outwardly with respect to an axial direction of the seal.

The peripheral wall may taper outwardly from a region of the second surface to the first surface with respect to an axial direction of the seal.

The first material and the friction-reducing material may comprise, consists of, or essentially consists of one or more polymers.

The friction-reducing material may comprise, consists of, or essentially consists of a fluoropolymer, and the friction-reducing material may comprise, consists of, or essentially consists of Polytetrafluoroethylene (PTFE).

In some embodiments, the first material and/or the friction-reducing material are flexible. In some embodiments, the first material and/or the friction-reducing material are resiliently deformable.

The first material may comprise, consists of, or essentially consists of an elastomer, and, may comprise, consists of, or essentially consists of rubber.

The layer of friction-reducing material may comprise a thickness of at least 0.005 inches (0.127mm) and may comprise at least 0.01 inches (0.254mm).

The layer of friction-reducing material may comprise a thickness of at most 0.02 inches (0.508mm) and may comprise at most 0.015 inches (0.381mm).

In some embodiments, the seal comprises an outer perimeter which is substantially circular in shape. In some embodiments, the seal is generally annular. In some embodiments, the seal comprises a central aperture. In some embodiments, the seal is a substantially solid component absent of a central aperture.

The seal may further comprise one or more locating formations configured to align the seal on a ventilator piston during mounting of the seal to a ventilator piston, and the one or more locating formations may comprise a protrusion or a depression.

In some embodiments, the deformable body comprises the locating formation(s).

The or each locating formation may follow a substantially arcuate path that subtends at least partially about the central axis of the seal, and the or each locating formation may follow a substantially annular path about the central axis of the seal.

In some embodiments, the locating formation(s) is disposed a first side of the seal and a second side of the seal, opposite to the first side, may comprise the layer of friction-reducing material. In some embodiments, the first side also comprises a layer of friction-reducing material.

In some embodiments, at least a part of the or each locating formation is integral with the deformable body and formed from the first material. In some embodiments, the or each locating formation comprises the friction-reducing material or a third material and is moulded to the deformable body. In some embodiments, the or each locating formation comprises a continuous annular protruding ridge. In some embodiments, the or each locating formation comprises a discontinuous series of protrusions.

The seal may further comprise a perimeter bead comprising a region of greater thickness of the seal extending around the circumference of the seal.

In some embodiments, the deformable body comprises the perimeter bead, and may be integrally formed in the deformable body. In some embodiments, the perimeter bead may be formed of the friction-reducing material.

The perimeter bead may comprise one or more strengthening elements, and the or each strengthening element may be annular.

In some embodiments, the or each strengthening element may be embedded within the deformable body. The or each strengthening element may comprise metal, such as steel.

The friction-reducing material may comprise a skin of material bonded to the deformable body. The friction-reducing material may comprises a skin of material moulded with the support body.

The seal may further comprise an integral structural fixed relative to the deformable body, and which may be bonded or moulded to the deformable body. The structural element may comprise a piston head. The structural element may be made of plastic or metal, for example, steel, aluminium or titanium. The structural element, such as a piston or piston head, may be embedded within the deformable body or may be mounted to a surface of the deformable body.

The seal may further comprise a further layer of material on a surface of the deformable body opposite to the layer of friction-reducing material. The further layer of material may comprise the friction-reducing material. The entire outer surface of the deformable body may be covered by the friction-reducing material.

Also provided is a ventilator comprising a piston chamber having an inner wall, and a piston comprising a piston head having a seal as described above secured to the piston, wherein the piston and seal are moveably received within the piston chamber and arranged such that the friction-reducing material of the seal is in slidable sealing contact with the inner wall of the piston chamber.

The seal may be deflected to fit within the piston chamber such that the perimeter bead exerts a radially outward biasing force urging a region of the friction-reducing material against the inner wall of the piston chamber.

The seal maybe deflected such that the peripheral wall portion of the seal lies parallel to and in contact with the inner wall of the piston chamber. The peripheral wall portion may be deflected such that a portion thereof is substantially flat.

Also provided is a method of manufacturing a seal for a ventilator piston as described above, the method comprising providing a portion of the first material; providing a layer of friction-reducing material; and compressing the first material and the friction-reducing material in a moulding tool to form the seal.

The method may further comprise providing a further layer of friction-reducing material; compressing the first material between the layers of friction-reducing material in a moulding tool to form the seal.

Compressing the first material and the friction-reducing material may permanently change the shape of the first material and the friction-reducing material.

The method may further comprise moulding or bonding a piston head to the seal.

### Brief Description of the Drawings

Embodiments of the invention will now be described, by way of example only, with reference to the accompanying drawings, in which:
**Figure 1** shows a schematic plan view of a first surface of a dual-material ventilator seal according to a first embodiment of the invention showing mainly a support body of the seal;
**Figure 2** shows a schematic plan view of a second surface of the dual-material ventilator seal of Figure 1 showing mainly a skin of the seal;
**Figure 3** shows a schematic cross-sectional view of the dual-material ventilator seal of Figures 1 and 2;
**Figure 4** shows a perspective view of a portion of the dual-material ventilator seal of Figures 1 to 3, looking towards the second surface at an angle;
**Figure 5** shows a perspective view of the dual-material ventilator seal of Figures 1 to 4, looking towards the first surface at an angle;
**Figure 6a** shows a schematic diagram of an example ventilator for use with the dual-material ventilator seal of Figures 1 to 5;
**Figure 6b** shows a schematic cross-sectional view of the dual-material ventilator seal of Figures 1 to 5, assembled with an example ventilator piston and piston chamber;
**Figure 7** shows a schematic plan view of the dual-material ventilator seal of Figures 1 to 6, assembled with an example ventilator piston, looking towards the first surface;
**Figure 8** shows a schematic plan view of a first surface of a dual-material ventilator seal according to a second embodiment of the invention showing mainly a support body of the seal without a locating formation;
**Figure 9** shows a schematic cross-sectional view of a part of a dual-material ventilator seal according to a third embodiment of the invention showing a strengthening element embedded within a bead of the seal;
**Figure 10** shows a schematic cross-sectional view of a part of a dual-material ventilator seal according to a fourth embodiment of the invention showing the skin only on a part of the second surface;
**Figure 11** shows a schematic cross-sectional view of a part of a dual-material ventilator seal according to a fifth embodiment of the invention showing a layer of friction-reducing material on both the first surface and the second surface;
**Figure 12** shows a schematic cross-sectional view of a dual-material ventilator seal according to a sixth embodiment of the invention showing an integral piston plate;
**Figure 13** shows a schematic cross-sectional view of a dual-material ventilator seal according to a seventh embodiment of the invention showing an integral piston plate at least partially enveloped by the body;

### Detailed Description

The scope of protection sought for various embodiments of the invention is set out by the independent claims. The embodiments and features, if any, described in the specification that do not fall under the scope of the independent claims are to be interpreted as examples useful for understanding various embodiments of the invention.

In the description and drawings, like reference numerals refer to like elements throughout.

There are a number of disadvantages typically found in known diaphragm ventilators. For example, some diaphragm ventilators experience irregular collapse and expansion of the diaphragm, resistance to piston movement, and fatigue of the diaphragm. Failure and resistance of the diaphragm can also cause large mechanical stresses. In some instances, a higher ventilator pressure can be required for providing breathable air to a patient's lungs. In some cases, when a higher pressure is applied, the bellows may simply inflate under the increased pressure rather than the increased pressure being applied to the breathable air supplied to the patient's lungs. All of the above can lead to ineffective or unreliable operation of the ventilator. An improved alternative to the diaphragms and bellows of the prior art is therefore required.

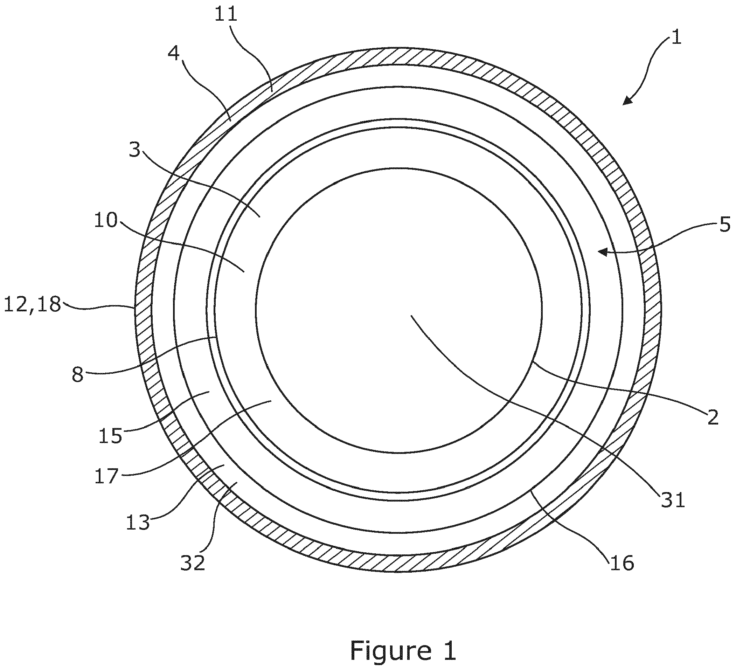

A dual-material ventilator seal according to a first embodiment is described herein with reference to Figures 1 to 7.

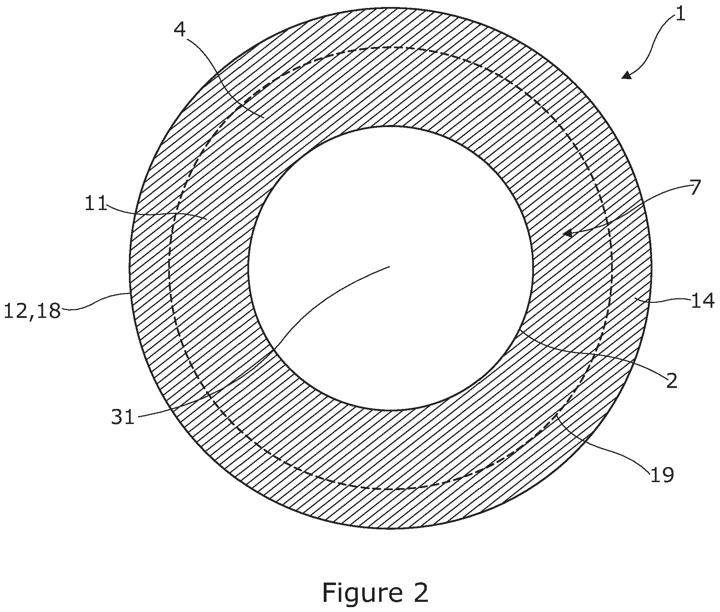

As illustrated in Figures 1 and 2, there is a ventilator seal 1 comprising a circular perimeter 12 and an aperture 2. Preferably the aperture 2 is provided in the centre such that the seal 1 is substantially annular in shape. The seal 1 comprises a substantially planar component having a laminar construction. The laminar construction of the seal 1 comprises a deformable body 3 comprising a first material 10 and a layer 4 of a friction-reducing 11 provided on the support body 3. The layer 4 of friction-reducing 11 is herein referred to as a skin 4.

The first material 10 and the friction-reducing 11 are different materials, such that the seal 1 is a dual-material ventilator seal. Preferably the first material 10 is suitable to provide structural support to the friction-reducing 11. Preferably both the first material 10 and the friction-reducing 11 are elastically deformable.

Preferably the first material 10 and the friction-reducing 11 are different types of polymer. More preferably the first material 10 is an elastomer and the friction-reducing 11 is a fluoropolymer. In some embodiments the friction-reducing 11 is Polytetrafluoroethylene (PTFE). In some embodiments the first material 10 is rubber.

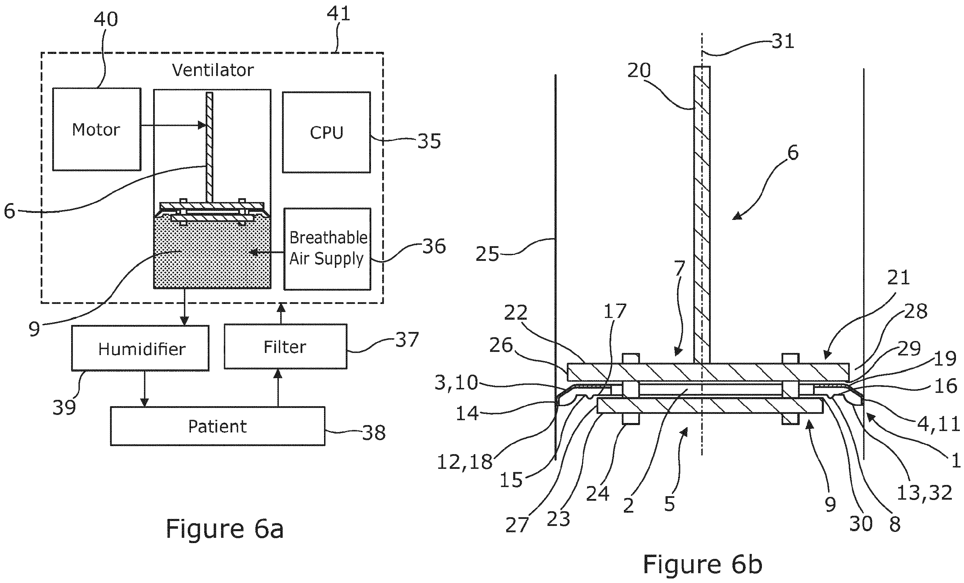

The seal 1 comprises opposing first and second surfaces. The seal 1 comprises a first surface 5 which is shown in Figure 1. The first surface 5 is disposed facing away from a piston 6, when installed and in use in a ventilator 41 (one example of the seal 1 assembled with a ventilator piston 6 is shown in Figure 6b).

The seal 1 also comprises a second surface 7, which is disposed oppositely to the first surface 5 and is shown in Figure 2. When assembled with the piston 6, the second surface 7 is disposed towards the piston 6 in use. The majority of the first surface 5 comprises a surface of the support body 3, and the majority of the second surface 7 comprises the skin 4. Preferably the skin 4 comprises substantially all of the second surface 7.

If the skin 4 is less than a certain thickness then there is a risk that it could be worn away during use to expose the underlying body 3 of first material 10. As the first material 10 does not comprise low friction properties to the same extent as the friction-reducing 11, or at all, the seal 1 would then not provide the slidable seal as required if the skin 4 is worn away. If the skin 4 is greater than a certain thickness then it may provide too much resistance to deformation and override the support properties of the body 3. Therefore, the skin 4 preferably comprises a thickness of between 0.005 inches (0.127mm) and 0.02 inches (0.508mm) to establish a balance between these two situations.

In an embodiment, both the first surface 5 and the second surface 7 comprise a portion of the skin 4.

The seal 1 further comprises a locating formation 8 on the first surface 5. The locating formation 8 is configured to locate the seal 1 on the piston 6 (shown in Figure 6b). As the locating formation 8 of the seal 1 shown in Figures 1 to 7 is disposed centrally, then the locating formation 8 is configured to centrally align the seal 1 on the piston 6 during assembly of the piston 6.

Advantageously aligning the seal 1 centrally on the piston helps to ensure that the seal 1 is located centrally within a piston chamber 9 (see Figures 6a and 6b) of a ventilator 41 of an embodiment within which the seal is to be used. This provides a uniform perimeter seal between the seal 1 and the piston chamber 9 which can avoid gas leakage and uneven wear, and therefore improve the performance, longevity and efficiency of the ventilating operation.

The locating formation 8 shown in Figure 1 is a continuous ridge which protrudes from the first surface 5 and is substantially annular in shape. The locating formation 8 is disposed closer to the outer perimeter 12 than a central longitudinal axis 31. However, this is dependent on the size of the piston 6 that the seal 1 is configured to be assembled to.

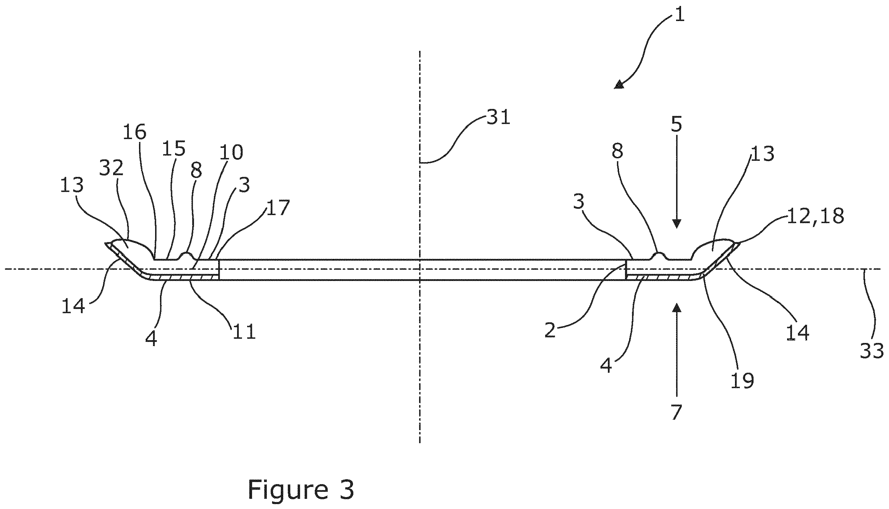

Preferably the locating formation 8 is integral with the support body 3 and formed from the first material 10. As illustrated best in Figure 3, the locating formation 8 is semi-circular or rounded in cross section. The locating formation 8 is disposed on the opposite side of the support body 3 to the skin 4.

As illustrated in Figure 3, the seal 1 comprises a bead 13 disposed proximate the perimeter 12 and a peripheral wall or sealing edge 14 extending between a planar and/or parallel region of the first and second surfaces 5, 7. The peripheral wall 14 may comprise part of the second surface 7 and may extend at a radially outer-most region of the planar portion of the second surface, towards the perimeter of the first surface. The bead 13 is configured to provide an outward biasing force on the sealing edge 14 during use of the seal in a ventilator 41, as will be described in more detail hereafter.

The bead 13 comprises a rounded thickening of the support body 3 comprised of the first material 10. The bead 13 comprises a tip 32, which is the part of the bead 13 that extends furthest from a horizontal central axis 33 of the substantially planar portion of the seal 1.

Between the bead 13 and the locating formation 8 is a first flange 15 of the support body 3. The bead 13 and the first flange 15 meet at a convergence point 16. Between the locating formation 8 and the aperture 2, or the central longitudinal axis 31, is a second flange 17 of the support body 3. Preferably the first flange 15 and the second flange 17 are substantially flat. Preferably the first flange 15 and the second flange 17 comprise the same thickness.

The height of the bead 13, from the convergence point 16 to the tip 32, is preferably greater than half the thickness of the first and/or second flange 15, 17. The height of the bead 13, from the convergence point 16 to the tip 32, is preferably greater than the amount the locating formation 8 protrudes from the support body 3.

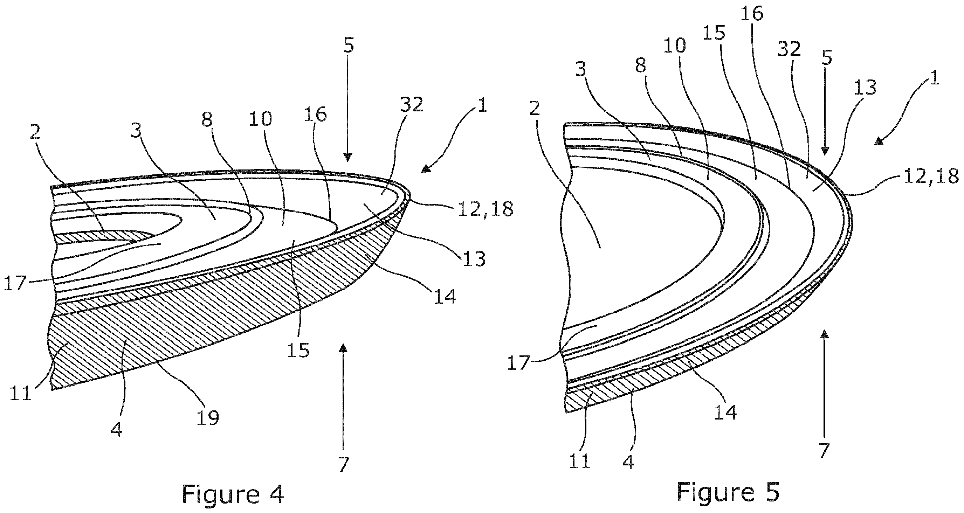

As illustrated in Figures 4 and 5, the peripheral wall 14 extends between regions of the first surface 5 and the second surface 7. The peripheral wall 14 is inclined or tapers inwardly towards the central longitudinal axis 31 as it extends from an outer edge 18 of the first surface 5 to an outer edge 19 of the planar region of the second surface 7.

Preferably the skin 4 extends over a portion of the peripheral wall 14. More preferably the skin 4 extends from the planar region of the second surface 7 and across the peripheral wall 14, at least to, and preferably entirely to, the outer edge 18 of the first surface 5.

Advantageously the peripheral wall 14 being covered with the skin 4 of friction-reducing means that it is the friction-reducing 11 which is in contact with the piston chamber 9 (shown in Figure 6b). As the friction-reducing 11 is a material which preferably exhibits low-friction properties, lubrication by means of an additive material is advantageously not required for reliable operation of the piston 6 whilst the friction-reducing 11 of the seal 1 is in contact with the piston chamber 9.

The skin 4 on the peripheral wall 14 may also be configured to deposit some of the friction-reducing 11 on an inner wall 25 of the piston chamber 9 during use. This assists in forming the seal and further enhancing low friction properties between the piston chamber 9 and the seal 1.

Figure 6a shows a diagram of an example ventilator 41. The ventilator 41 comprises a motor 40 which drives a piston 6 in a piston chamber 9. A breathable air supply 36 supplies breathable air to the piston chamber 9 to be pushed into a patient's lungs 38 by the piston 6. The amount of breathable air pushed into a patient's lungs 38 is controlled by a CPU 35.

Before the breathable air enters the patient's lungs 38 it passes through a humidifier 39 which warms and moistens the air. In some embodiments as air is exhaled from the patient's lungs 38 it passes through a filter 37 and away from the patient 38.

It can be appreciated that there are many types of ventilators in which the seal 1 of the present invention can be used, and the diagram provided in Figure 6a is a simplified example.

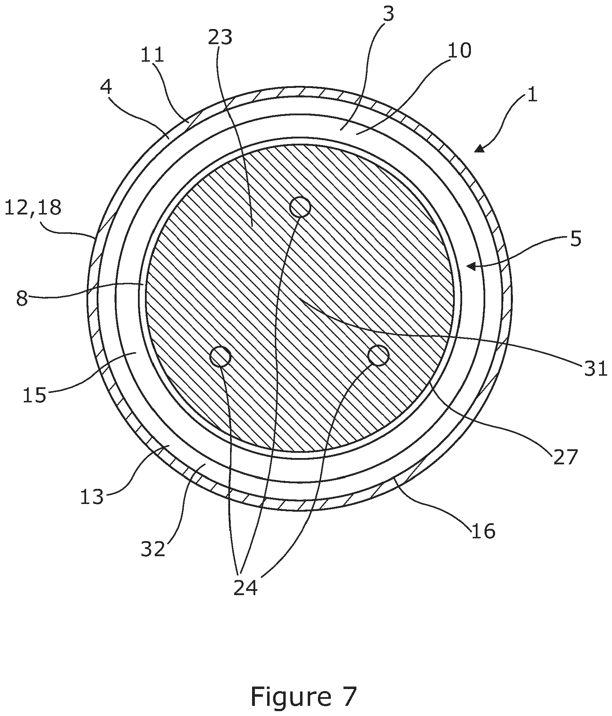

Figures 6b and 7 show the seal 1 assembled to a piston 6 of a ventilator 41 in more detail. The piston 6 comprises a piston rod 20 and a piston head 21. The piston head 21 comprises a pair of clamp plates, a first clamp plate 22 and a second clamp plate 23.

The first clamp plate 22 is attachable to the second clamp plate 23 via attachment means 24. In the embodiment shown in Figures 6b and 7, the attachment means are threaded screws 24 which engage with threaded screw holes in the clamp plates 22, 23. However, it can be appreciated that the clamp plates 22, 23 can be attached by any known attachment means 24.

The first clamp plate 22 comprises a mating face 29 which lies against the second surface 7 of the seal 1 when assembled as shown in Figure 6b. The second clamp plate 23 comprises a mating face 30 which lies against the first surface 5 of the seal 1 when assembled as shown in Figure 6b.

The outer perimeter 12 of the seal 1 is configured to extend further from the central longitudinal axis 31 than an outer edge 26, 27 of either the first clamp plate 22 or the second clamp plate 23 respectively. This is so that in use, between the piston head 21 and an inner wall 25 of the piston chamber 9 there is a gap 28, to avoid the piston head 21 from contacting the inner wall 25 as it moves up and down in the piston chamber 9.

The seal 1 is compressible between the first clamp plate 22 and the second clamp plate 23. Compressing the seal 1 between the clamp plates 22, 23 provides a gas impermeable seal at the mating faces 29, 30 of the seal 1 and the clamp plates 22, 23.

To effectively compress the seal 1, both the first clamp plate 22 and the second clamp plate 23 must be greater in size than the aperture 2 in the seal 1. In the embodiments shown and described in relation to Figures 1 to 7, the aperture 2 of the seal 1 is configured to have a diameter which is less than the diameter of the first clamp plate 22 and less than the diameter of the second clamp plate 23.

As previously described in relation to Figure 1, the locating formation 8 serves to locate the seal 1 on the piston 6. Particularly, the second clamp plate 23 is located by the locating formation 8 of the seal 1 to ensure that the piston head 21, and therefore the piston rod 20 are located centrally in the piston chamber 9.

Preferably, the area enclosed by the locating formation 8 is the same as the area of the mating face 30 of the second clamp plate 23. This is such that at least a part of the locating formation 8 is configured to abut the outer edge 27 of the second clamping plate 23 when the seal 1 is assembled with the piston 6.

When installed in the piston chamber 9, the peripheral wall 14 of the seal 1 is deformed radially inwardly towards the longitudinal central axis 31. This causes a circumferential constriction of the bead 13 and deflects the bead 13 inwardly towards the second flange 17. In this configuration, the resilience of the elastically-deformed bead 13 provides an outward force to bias the peripheral wall 14 of the seal 1 against the inner wall 25 of the piston chamber 9 to provide a slidable seal. The slidable seal between the seal 1 and the inner wall 25 of the piston chamber 9, enables gas to be pushed out of the chamber 9 and into a patient's lungs by the piston 6.

The seal 1 is manufactured by providing the body 3 of first material 10 and the skin 4 of friction-reducing 11, in a pre-prepared form which is not their final form. The pre-prepared body 3 and the pre-prepared skin 4 are then compressed in a moulding tool to such that the material of the respective bodies flows under pressure in the mould into the shape of the seal 1 in its final form. The pre-prepared form of the body 3 or skin 4 could be a block, ring, tube or any alternatively shaped part.

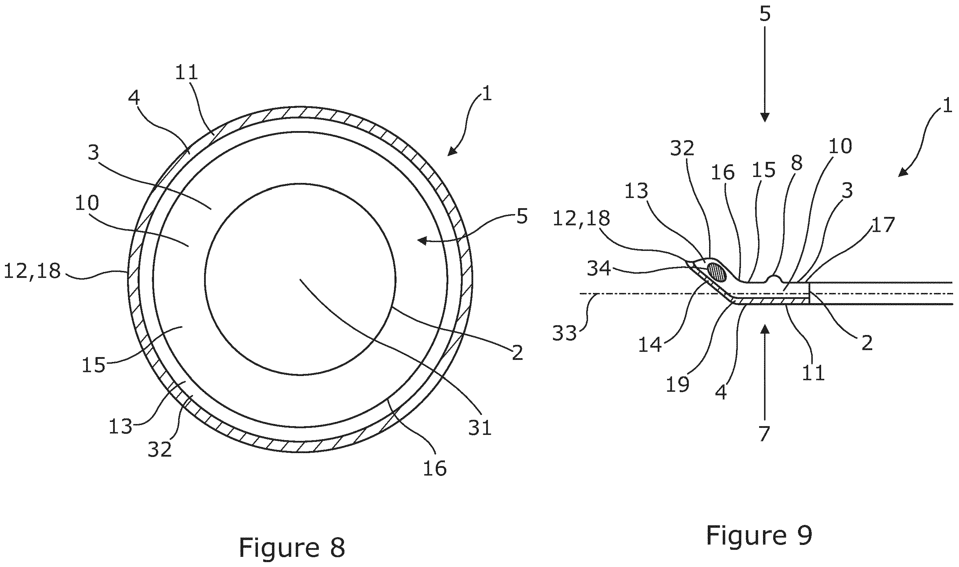

Figure 8 shows a dual-material ventilator seal 1 according to a second embodiment. The dual-material ventilator seal 1 has similar features to the seal 1 of the first embodiment described above in relation to Figures 1 to 7, with like features retaining the same reference numerals. A difference is that the seal 1 does not comprise a locating formation 8.

In this embodiment, the piston 6 may comprise a locating means, for example a protrusion in the centre of one of the clamp plates 22, 23 which engages with the aperture 2 of the seal 1 to locate the seal on the piston 6. Alternatively the seal 1 maybe measured and installed precisely in the centre of the piston 6 during the assembly process, without a separate locating means.

As there is no locating formation 8, the first flange 15 and the second flange 17 are integral. In some embodiments they can both be considered the first flange 15.

Figure 9 shows a cross-section of a part of a dual-material ventilator seal 1 according to a third embodiment. The dual-material ventilator seal 1 has similar features to the seal 1 of the previous embodiments described above in relation to Figures 1 to 8, with like features retaining the same reference numerals. A difference of the third embodiment is that the seal 1 comprises an annular strengthening element 34 embedded within the material of the support body 3.

The bead 13 provides a stiffening effect which resists deformation when fitted within a piston chamber 9 of a ventilator 41, and so urges the sealing edge 14 into enhanced sealing contact with the inner wall of the piston chamber 9. The strengthening element 34 provides improved strength to the bead 13 and assists in resisting deformation and holding the sealing edge 14 into enhanced sealing contact with the inner wall of the piston chamber 9.

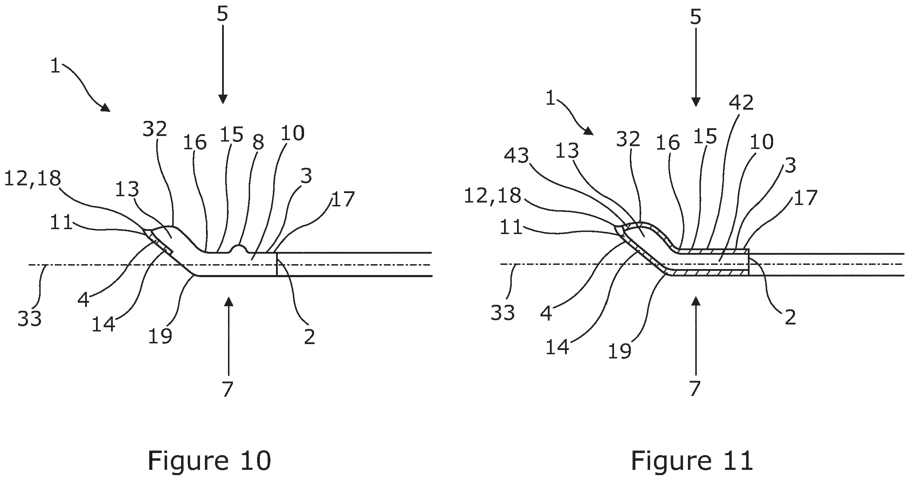

Figure 10 shows a schematic cross-sectional view of part of a dual-material ventilator seal 1 according to a fourth embodiment of the invention. The dual-material ventilator seal 1 has similar features to the seal 1 of the previous embodiments described above in relation to Figures 1 to 9, with like features retaining the same reference numerals. A difference of the fourth embodiment is that the skin 4 of friction-reducing 11 is provided only on a part of the peripheral wall 14.

As a minimum, the skin 4 is required only on the part of the peripheral wall 14 which contacts the inner wall 25 of the piston chamber 9 in use. This is because it is the friction-reducing 11 which forms the slidable seal between the seal 1 and the piston chamber 9, and only a part of the peripheral wall 14 may contact the inner wall 25 of the piston chamber 9 in use.

When manufacturing the seal 1 of the fourth embodiment, the pre-prepared form of the skin 4 may be an annular ring. This annular ring can be compressed with a pre-prepared body 3 by a moulding tool (not shown) to form a final form seal 1 such as that shown in Figure 10.

Figure 11 shows a schematic cross-sectional view of a dual-material ventilator seal 1 according to a fifth embodiment of the invention. The dual-material ventilator seal 1 has similar features to the seal 1 of the previous embodiments described above in relation to Figures 1 to 10, with like features retaining the same reference numerals. A difference of the fifth embodiment is that the seal 1 comprises a further layer 42 of friction-reducing 11.

The further layer 42 (or further skin 42) of friction-reducing 11 is provided on the opposing surface of the seal 1 to the skin 4. In the embodiment illustrated in Figure 11, the skin 4 is provided on the second surface 7 and the further skin 42 is provided on the first surface 5. The further skin 42 is preferably thinner than the skin 4, for example the skin 4 comprises a thickness of 0.01 inches (0.254mm) and the further skin 42 comprises a thickness of 0.005 inches (0.127mm).

Between the skin 4 and the further skin 42 there may be a seam 43, where the two meet. This seam 42 is situated away from the peripheral wall 14, and radially inwardly of the inner wall of the piston chamber in use, such that the seam 42 does not come into contact with the inner wall 25 of the piston chamber 9. This is because the seam 42 could reduce the effectiveness of the slidable seal. Advantageously the further skin 42 can provide protection to the underlying body 3 of first material 10.

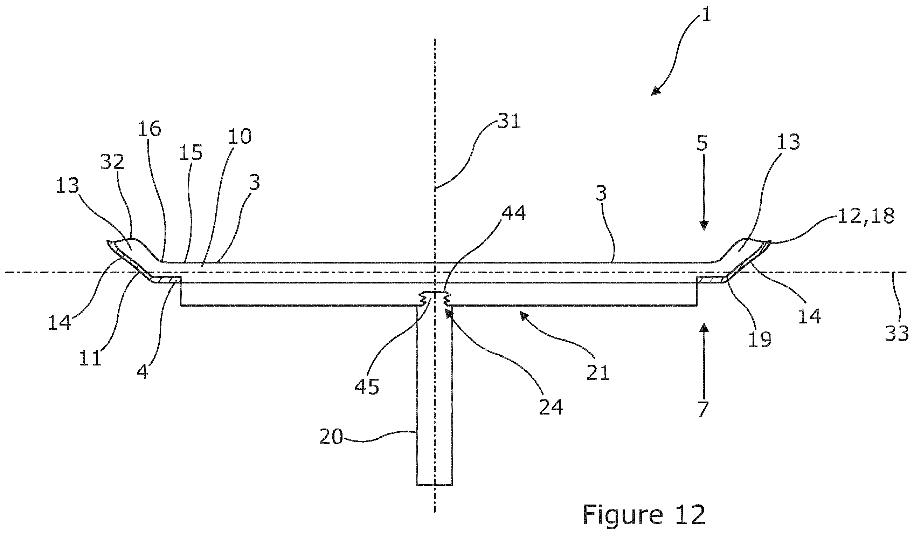

Figure 12 shows a schematic cross-sectional view of a dual-material ventilator seal 1 according to a sixth embodiment of the invention. The dual-material ventilator seal 1 has similar features to the seal 1 of the previous embodiments described above in relation to Figures 1 to 11, with like features retaining the same reference numerals. A difference of the sixth embodiment is that the seal 1 comprises an integral structural element, in this exemplary embodiment in the form of a piston head 21.

The piston head 21 comprises an attachment means 24, which in the sixth embodiment comprises an aperture with a threaded bore 44 to engage with a piston rod 20 comprising a mating portion 45 with a screw thread at one end.

The skin 4 extends from the perimeter 12 of the seal 1 up to the integral piston head 21 and the piston head 21 may be bonded, moulded or attached to the body 3.

The piston head 21 maybe bonded or moulded to the seal 1 as part of the manufacturing process. The piston head 21 comprises a material which is different from the first material 10 and the friction-reducing 11 of the body 3 and the skin 4. Preferably the piston head 21 is made from metal, for example steel. Therefore, during the manufacturing of the seal 1 of the sixth embodiment, three different pre-prepared materials are compressed in the moulding tool at the same time.

Advantageously having an integral piston head 20 reduces the number of components involved in the assembly of the ventilator. This can reduce the weight of the piston 6 and improve the ease of assembly and manufacture.

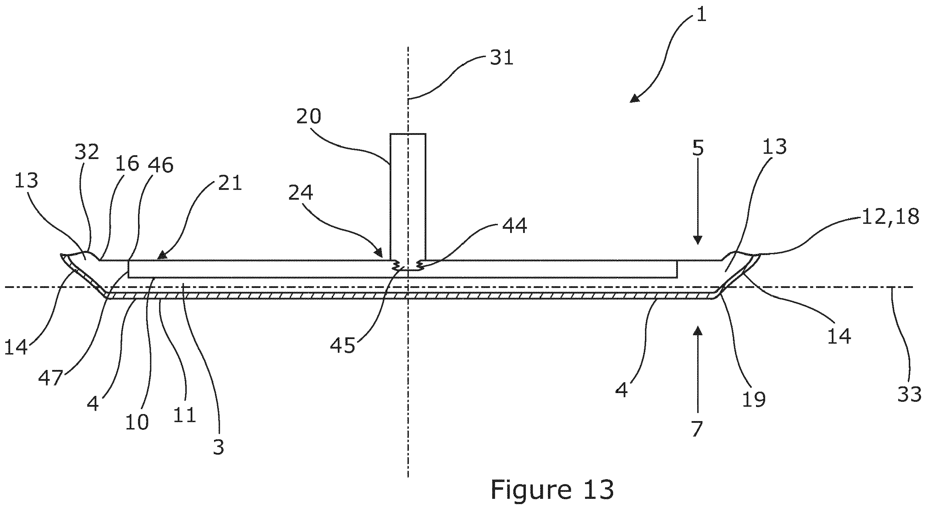

Figure 13 shows a schematic cross-sectional view of a dual-material ventilator seal 1 according to a seventh embodiment of the invention. The dual-material ventilator seal 1 has similar features to the seal 1 of the previous embodiments described above in relation to Figures 1 to 12, with like features retaining the same reference numerals. One difference of the seventh embodiment is that the piston head 21 is at least partially enveloped by the body 3. A further difference of the seventh embodiment is that the seal 1 does not comprise a central aperture.

The piston head 20 comprises the same attachment means 24 as the sixth embodiment and may be manufactured in the same way, providing similar advantages. In the embodiment shown in Figure 13, the piston head 21 only contacts the body 3 and is separate from the skin 4. Furthermore, the piston head 21 and the body 3 form a substantially flat first surface 5 comprising an annular seam 46 in the first surface 5. The annular seam 46 is formed by edges 47 of the piston head 21 being at least partially embedded in the body 3.

Embedding the piston head 21 within the body 3 provides a larger surface area for bonding or moulding the two together. The piston head 21 may also provide additional support to the body 3.

The seal 1 does not comprise the central aperture 2, and is therefore considered substantially solid rather than annular in shape. Any of the previously-described embodiments may comprise a substantially solid seal 1. As illustrated in Figure 13, the skin 4 extends entirely within the boundaries of the perimeter 12, however other configurations can be envisaged, for example only part of the second surface 7 may comprise the skin 4.

It will be appreciated from the above that the seal, and ventilator comprising such a seal, of the present invention provides a cylinder and seal with substantially no capacity to expand and deflect if the ventilator is required to operate to deliver elevated breathable air pressure to a patient. Thereby, an above-mentioned problem of bellows or diaphragm-type ventilators is avoided.

Where the term 'layer' is referred to throughout, this is intended to mean a full layer, or part of a layer. For example, the skin 4 i.e. the layer of friction-reducing 11, can be ring shaped, circular, rounded or alternatively shaped to suit the piston chamber 9. The 'layer' may comprise at least one aperture.

Where the 'centre' of the seal 1 is referred to throughout, this is intended to mean the centre with respect to a central axis 31 in an axial direction of the seal. Said central axis 31 can be seen in Figure 3.

It can be appreciated that compatible features of the above described embodiments can be combined and substituted to form a seal 1 that falls within the scope of the present disclosure.

Many variants of the example embodiments are described above and discussed below. The skilled person will be aware of further variants and modifications that may be made to the embodiments described herein.

In the above described embodiments the seal 1 comprises a circular perimeter 12. However, in alternative embodiments intended within the scope of the present disclosure, the outer perimeter 12 can be any shape which compliments the shape of the piston chamber 9. For example, if the piston chamber 9 is polygonal then the outer perimeter 12 of the seal 1 is polygonal. It can be appreciated that the outer perimeter may be, for example but not limited to, polygonal, square, triangular or elliptical.

In the above described embodiments the seal 1 comprises an aperture 2. However, in alternative embodiments intended within the scope of the present disclosure, the seal 1 is a solid component absent of any apertures 2.

In the above described embodiments the aperture 2 is provided in the centre of the seal 1, such that the seal 1 is substantially annular in shape. However, in alternative embodiments intended within the scope of the present disclosure, the seal 1 comprises an aperture which is disposed off-centre from an axial direction of the seal 1. For example, the seal 1 comprises a plurality of apertures 2 disposed substantially centrally with respect to an axial direction of the seal.

In the above described embodiments the seal 1 comprises a support body 3 comprising a first material 10. It can be appreciated that the support body 3 is made entirely of the first material 10. Alternatively it can be appreciated that the support body 3 is made mainly of the first material 10. In some embodiments the support body 3 is made of the first material 10 and at least one other material that is different to the first material 10.

In the above described embodiments the first surface 5 is disposed facing away from a piston 6 when installed and in use in a ventilator 41 and an opposing second surface 7. However, in alternative embodiments intended within the scope of the present disclosure, the seal 1 can be configured to be installed such that the first surface 5 is disposed facing towards the piston 6 in use.

In the above described embodiments the majority of the first surface 5 comprises the support body 3, and the majority of the second surface 7 comprises the skin 4. However, in alternative embodiments intended within the scope of the present disclosure, the first surface 5 comprises the skin 4 and the second surface 7 comprises the support body 3. In some embodiments the skin 4 substantially covers the first surface 5. The skin 4 may also entirely cover the support body 3 on both sides thereof, to effectively provide a seal having a three layer construction, namely, viewed in cross-section, a support body 3 sandwiched between skin layers 4 on opposite surfaces of the support body 3.

In the above-described embodiments the skin 4 on the peripheral wall 14 maybe configured to deposit some of the friction-reducing 11 on an inner wall 25 of the piston chamber 9. In addition, in some embodiments a low friction coating (not shown) is applied on the inner wall 25 of the piston chamber 9. This low friction coating (not shown) can be sprayed or alternatively applied during manufacture or assembly of the ventilator. Advantageously this assists the seal 1 to form a slidable seal with the inner wall 25 of the piston chamber 9. It may also reduce wear of the skin 4 on the peripheral wall 14.

In the above described embodiments the locating formation 8 is a continuous ridge which protrudes from the second surface 5. However, in alternative embodiments intended within the scope of the present disclosure, the locating formation 8 can be a discontinuous ridge, an alternatively shaped protrusion, a groove or a series of tabs to locate the piston in the centre of the seal 1.

In the above-described embodiments the locating formation 8 is integral with the support body 3 and formed from the first material 10. However, in alternative embodiments intended within the scope of the present disclosure, the locating formation 8 can be formed of the friction-reducing 11 or a third material and moulded to the second surface 5.

In the above-described embodiments the locating formation 8 is semi-circular or rounded in cross section. However, in alternative embodiments intended within the scope of the present disclosure, the locating formation 8 maybe polygonal, or comprise a receiving portion, for example a C shape cross section, or a T shape cross section, for receiving the second clamp plate 23 of the piston 6.

Some of the above-described embodiments include a locating formation 8. However, it can be appreciated that the seal 1 of any embodiment described herein can be modified to include or remove the locating formation 8.

In the above-described embodiments the bead 13 is a rounded thickening of the support body 3 and is formed from the first material 10. However, in alternative embodiments intended within the scope of the present disclosure, the bead 13 can be formed of the friction-reducing 11 or a third material and moulded or bonded to the second surface 5. For example, the bead 13 may comprise a strengthening element bonded on, or embedded within the material of the support body 3. Preferably the strengthening element is annular in shape. Such an embodiment may comprise a strengthening element comprising a metallic ring, a plastic ring, or a ring of other material. The bead 13 provides a stiffening effect which resists deformation when fitted within a piston chamber 9 of a ventilator 41, and so urges the sealing edge 14 into enhanced sealing contact with the inner wall of the piston chamber 9. The smooth low-friction nature of the surface of the skin 4 also enhances the sealing performance of the seal 1 against the inner wall of the piston chamber 9.

In the above-described embodiments the first flange 15 and the second flange 17 are substantially flat. However, in alternative embodiments intended within the scope of the present disclosure, one or both of the first flange 15 and the second flange 17 may comprises further ridges, grooves or protrusions to provide friction or facilitate the deformation of the peripheral walls 14 when installed in the piston chamber 9.

In the above-described embodiments the first flange 15 and the second flange 17 are substantially flat. However, in alternative embodiments intended within the scope of the present disclosure, one or both of the first flange 15 and the second flange 17 may comprise a slope towards or away from the central longitudinal axis 31. In some embodiments, the first flange 15 and the second flange 17 slope in the same direction, in alternative embodiments the first flange 15 and the second flange 17 slope towards each other, or away from each other. In some embodiments the first flange 15 and/or the second flange 17 comprises a thicker portion and slope away from the thicker portion towards the perimeter 12 and the central axis 31 respectively.

In the above-described embodiments the first flange 15 and the second flange 17 comprise the same thickness. However, in alternative embodiments intended within the scope of the present disclosure, the thickness of the first flange 15 and the second flange 17 may be different. Furthermore, the thickness of the first flange 15 and/or the second flange 17 may not be consistent.

In the above-described embodiments the height of the bead 13, from the convergence point 16 to the tip 32, is greater than half the thickness of the first and/or second flange 15, 17. However, in alternative embodiments intended within the scope of the present disclosure, the height of the bead 13 is greater than the thickness of the first and/or second flange respectively 15, 17.

In the above-described embodiments a pre-prepared body 3 and a pre-prepared skin 4 are compressed in a moulding tool to shape the seal 1 into its final form. However, in alternative embodiments intended within the scope of the present disclosure, the support body 3 is moulded and the skin 4 is separately bonded to the support body 3.

In the above-described embodiments only a part of the peripheral wall 14 contacts the inner wall 25 of the piston chamber 9 in use. However, in alternative embodiments intended within the scope of the present disclosure, the whole of the peripheral wall 14 contacts the inner wall 25 of the piston chamber 9 in use.

In the above-described embodiments there is a seam 43 between the skin 4 and the further skin 42. However, in alternative embodiments intended within the scope of the present disclosure, the skin 4 and the further skin 42 are integral with one another such that there is no seam 43.

In the above-described embodiments the further skin 42 is thinner than the skin 4. However, in alternative embodiments intended within the scope of the present disclosure, the skin 4 and the further skin 42 may comprise the same thickness.

In the above-described embodiments the further skin 42 is made of the friction-reducing 11. However, in alternative embodiments intended within the scope of the present disclosure, the further skin 42 may be made of a different material which provides protective properties for the underlying body 3 and may not comprise low friction properties.

In the above-described embodiments the skin 4 extends from the perimeter 12 of the seal 1 up to the integral piston plate 20 and the piston plate 20 may be bonded, moulded or attached to the body 3. However, in alternative embodiments intended within the scope of the present disclosure, the skin 4 may extend past the edges of the piston plate 20. In some embodiments the piston plate 20 maybe bonded, moulded or attached to the skin 4. In other embodiments the skin 2 does not extend to the edges of the piston plate 20. In some embodiments the piston plate 20 does not contact the friction-reducing 11 at all.

In the above-described embodiments the piston head 20 and the body 3 form a substantially flat first surface 5. However, in alternative embodiments intended within the scope of the present disclosure, the piston head 20 may protrude from the body 3, even if partially embedded within the body 3.

In the above-described embodiments the piston head 20 is embedded in the body 3 such that the edges of the piston head 47 are at least partially enclosed by the body 3. However, in alternative embodiments intended within the scope of the present disclosure, the edges 47 of the piston head 20 are not covered or enclosed by the body and there exists a gap between the edges 47 of the piston head 20 and the bead 13 of the seal 1.

In the above-described embodiments the seal 1 comprises the skin 4 and the body 3, and in some cases the skin 4, further skin 42 and the body 3. However, in alternative embodiments intended within the scope of the present disclosure, the seal 1 may comprise further layers of alternative materials or the same materials, as already described herein.

In some embodiments the seal 1 is entirely wrapped by the skin 4 and/or further skin 42. In other embodiments the seal 1 is partially wrapped by the skin 4 and/or further skin 42. In some embodiments the whole of the second surface 7 is covered by the skin 4 and/or further skin 42. In some embodiments the whole of the first surface 5 is covered by the skin 4 and/or further skin 42. In some embodiments less than half of the first surface 5 is covered by the skin 4 and/or further skin 42. In some embodiments less than half of the second surface 7 is covered by the skin 4 and/or the further skin 42.

In each above described embodiments the piston 6 is arranged with the seal 1 such that the piston rod 20 is shown to be disposed on either the side of the first surface 5 or the second surface 11 of the seal 1. However it can be appreciated that any of the above described embodiments can be modified such that the piston 6 is arranged with the seal 1 such that the piston rod 20 is disposed on the other side from the first surface 5 or second surface 7.

In the above described embodiments the seal 1 is for use with a ventilator piston 6. However, it can be appreciated that the seal 1 is suitable for other applications and industries in which a slidable gas impermeable seal is required between a piston 6 and a piston chamber 9. For example but not limited to, food production, gas processing or other air systems.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described example embodiments and/or the dependent claims with the features of the independent claims, and not solely the combinations explicitly set out in the claims.

## Claims

1. A seal for a ventilator piston, the seal configured to be secured to a ventilator piston and received within a piston chamber of a ventilator to provide a slidable seal against an inner wall of the piston chamber, the seal comprising:
a deformable body comprising a first material; and
a layer of a friction-reducing material provided on at least a portion of the deformable body and configured to provide slidable sealing contact with the inner wall of the piston chamber;
wherein the first material and the friction-reducing material are different.

2. A seal according to claim 1, wherein the deformable body comprises a substantially planar component.

3. A seal according to claim 1 or claim 2, wherein the seal comprises a laminar construction and opposing first and second surfaces, wherein the layer of friction-reducing material substantially covers at least one or part of the first and second surfaces; and/or the seal further comprises a peripheral wall and the layer of friction-reducing material extends at least partially over the peripheral wall, and optionally wherein the peripheral wall tapers outwardly from a region of one of the first surface and the second surface, to the other of the first surface and the second surface.

4. A seal according to any of claims 1 to 3, wherein the first material and the friction-reducing material comprise, consists of, or essentially consists of one or more polymers; and/or
wherein the friction-reducing material comprises, consists of, or essentially consists of a fluoropolymer, and preferably, wherein the friction-reducing material comprises, consists of, or essentially consists of Polytetrafluoroethylene (PTFE); and/or
wherein the first material comprises, consists of, or essentially consists of an elastomer, and, preferably, wherein the first material comprises, consists of, or essentially consists of rubber.

5. A seal according to any of claims 1 to 4, wherein the layer of friction-reducing material comprises a thickness of at least 0.005 inches (0.127mm) and, preferably, at least 0.01 inches (0.254mm) and/or wherein the layer of friction-reducing material comprises a thickness of at most 0.02 inches (0.508mm) and, preferably, at most 0.015 inches (0.381mm).

6. A seal according to any of claims 1 to 5, further comprising one or more locating formations configured to align the seal on a ventilator piston during mounting of the seal to a ventilator piston, and preferably, wherein the one or more locating formations comprises a protrusion or a depression; and/or
wherein the or each locating formation follows a substantially arcuate path that subtends at least partially about the central axis of the seal, and preferably the or each locating formation follows a substantially annular path about the central axis of the seal.

7. A seal according to any of claims 1 to 6, further comprising a perimeter bead comprising a region of greater thickness of the seal extending around the circumference of the seal.

8. A seal according to claim 7, wherein the perimeter bead comprises one or more strengthening elements, and preferably, the or each strengthening element is annular.

9. A seal according to any of claims 1 to 8, wherein the friction-reducing material comprises a skin of material bonded to or moulded with the deformable body.

10. A seal according to any of claims 1 to 9, further comprising a piston head fixed relative to the deformable body and, preferably, the piston head is embedded within the deformable body or is mounted to a surface of the deformable body.

11. A seal according to any of claims 1 to 10, further comprising a further layer of material on a surface of the deformable body opposite to the layer of friction-reducing material, optionally wherein the further layer of material comprises the friction-reducing material and/or wherein the entire outer surface of the deformable body is covered by the friction-reducing material.

12. A ventilator comprising a piston chamber having an inner wall, and a piston comprising a piston head having a seal according to any of claims 1 to 11 secured to the piston, wherein the piston and seal are moveably received within the piston chamber and arranged such that the friction-reducing material of the seal is in slidable sealing contact with the inner wall of the piston chamber.

13. A ventilator according to claim 12, when dependent on claim 7, wherein the seal is deflected to fit within the piston chamber such that the perimeter bead exerts a radially outward biasing force urging a region of the friction-reducing material against the inner wall of the piston chamber.

14. A method of manufacturing a seal for a ventilator piston according to any of claims 1 to 11 comprising:
providing a portion of the first material;
providing a layer of friction-reducing material;
compressing the first material and the friction-reducing material in a moulding tool to form the seal.

15. A method of manufacturing a seal according to claim 14 the method further comprising:
providing a further layer of friction-reducing material;
compressing the first material between the layers of friction-reducing material in a moulding tool to form the seal; and/or
moulding or bonding a piston head to the seal; and/or
wherein compressing the first material and the friction-reducing material permanently changes the shape of the first material and the friction-reducing material.
